# EUROPÄISCHE PATENTSCHRIFT

(11) **EP 2 332 197 B1**
(45) Veröffentlichungstag und Bekanntmachung des Hinweises auf die Patenterteilung: **25.12.2013**
(21) Anmeldenummer: 09778610.7
(22) Anmeldetag: 18.09.2009
(51) Int. Cl.: H01M 2/10, A61M 1/14

(54) **HALTERUNG ZUM LÖSBAREN AUFNEHMEN EINER EINRICHTUNG AN EINER MEDIZINISCHEN VORRICHTUNG UND ENTSPRECHENDE MEDIZINISCHE VORRICHTUNG**
HOLDER FOR THE DETACHABLE ACCOMMODATION OF A DEVICE ON A MEDICAL APPARATUS AND CORRESPONDING MEDICAL APPARATUS
SUPPORT DE RÉCEPTION LIBÉRABLE D'UN DISPOSITIF SUR UN SYSTÈME MÉDICAL ET SYSTÈME MÉDICAL CORRESPONDANT

(30) Priorität: 22.09.2008 DE 102008048263
(43) Veröffentlichungstag der Anmeldung: 15.06.2011
(73) Patentinhaber: Fresenius Medical Care Deutschland GmbH, 61352 Bad Homburg (DE)
(72) Erfinder: FÖRGER, Jens, 35789 Laubuseschbach (Weilmünster) (DE); OESTERREICH, Stefan, 61267 Neu-Anspach (DE)
(74) Vertreter: Bobbert & Partner
(86) Internationale Anmeldenummer: PCT/EP2009/006763
(87) Internationale Veröffentlichungsnummer: WO 2010/031572

(56) Entgegenhaltungen:
- EP-A- 0 950 535
- DE-U1-202007 016 137
- JP-A- 2002 367 578
- JP-A- 2003 007 276
- US-B1- 6 181 380

## Beschreibung

Die vorliegende Erfindung betrifft eine Halterung zum lösbaren Aufnehmen sowohl der Halterung als auch einer Einrichtung, welche in einem Inneren der Halterung Platz finden kann, mit den Merkmalen des Anspruchs 1. Sie betrifft ferner eine medizinische Vorrichtung gemäß Anspruch 14.

Eine Reihe von aus der Praxis bekannten Vorrichtungen, insbesondere medizinischen Vorrichtungen, weisen eine - beispielsweise in Aussparungen oder Vertiefungen hiervon - lösbar aufnehmbare Einrichtung auf. Diese kann in einen Abschnitt der medizinischen Vorrichtung eingesteckt oder eingeschoben und entsprechend wieder von der medizinischen Vorrichtung getrennt werden. So sind beispielsweise in Dialysemaschinen integrierte Akkus oder Batterien austauschbar eingesteckt. Diese Spannungsquellen müssen regelmäßig ausgetauscht werden und sind daher auf einfache Weise einsetzbar und wieder entnehmbar angeordnet. Die Austauschbarkeit ist regelmäßig auch bei weiteren Einrichtungen, welche nicht der Stromversorgung der medizinischen Vorrichtung dienen, erforderlich.

Aus der DE 20 2007 016137 U1 ist ein schnelllösbarer Batteriebehälter mit einem Gestell, einem Behälter mit Arretierelementen, einer Batterieeinheit und einem Befestigungselement bekannt.

Aus der JP 2002 367578 A ist ein Batteriegehäuse in einem Flüssigkeitsbehälter bekannt.

Aus der US 6 181 380 B1 ist eine elektronische Bildaufnahmevorrichtung mit einem Objektivschutzmechanismus bekannt.

Aus der JP 2003 007276 A ist eine Befestigungsvorrichtung für einen Ausdehnungskörper bekannt.

Aus der EP 0 950 535 A ist ein Aufzeichnungsgerät und Batteriehaltemechanismus zum Gebrauch in diesem Gerät bekannt.

Aufgabe der vorliegenden Erfindung ist es, eine weitere Halterung zum lösbaren Befestigen oder Vorsehen einer Einrichtung, insbesondere einer Spannungsquelle, an einer Vorrichtung, insbesondere einer medizinischen Vorrichtung, vorzuschlagen.

Die erfindungsgemäße Aufgabe wird gelöst durch eine Halterung mit den Merkmalen des Anspruchs 1.

So wird erfindungsgemäß eine Halterung zum lösbaren Aufnehmen der Halterung an einer Vorrichtung, insbesondere einer medizinischen Vorrichtung, vorgeschlagen. Die erfindungsgemäße Halterung ist ferner geeignet zur Aufnahme bzw. zum Aufnehmen einer Einrichtung in ihrem Inneren in einem Gebrauchszustand der Halterung. Die Halterung kann dabei auf beliebige Weise ausgestaltet sein, um die Einrichtung im Inneren der Halterung aufzunehmen oder eingesetzt zu bekommen. Insbesondere ist die Halterung ausgestaltet, um eine Einrichtung vorbestimmter Abmessungen, Geometrie, Größe, Form oder dergleichen aufzunehmen. Insofern können Halterung und Einrichtung aufeinander abgestimmt sein. Die Halterung ist vorgesehen, die Einrichtung an der medizinischen Vorrichtung lösbar zu befestigen. Dieser Zustand wird durch den hier verwendeten Begriff "gebrauchsgemäß" ausgedrückt oder umschrieben.

Die erfindungsgemäße Halterung weist wenigstens zwei Seitenteile auf, nämlich ein erstes Seitenteil und ein zweites Seitenteil.

Dabei ist wenigstens das zweite Seitenteil oder ein Abschnitt hiervon von einer ersten Stellung in eine zweite Stellung überführbar. Die folgenden Ausführungen können ungeschmälert auf weitere Seitenteile als nur das zweite übertragen oder erstreckt werden. So können die folgende Ausführungen beispielsweise sowohl auf das erste als auch auf das zweite Seitenteil zutreffen.

Die erfindungsgemäß als Seitenteile bezeichneten Elemente der Halterung können beispielsweise als vertikale Begrenzungen des Inneren der Halterung ausgestaltet sein. Dabei kann u.a. auch eine Stirnseite im Rahmen der vorliegenden Erfindung als Seitenteil verstanden werden.

Unter einem lösbaren Aufnehmen der Halterung an einer medizinischen Vorrichtung wird insbesondere ein Einstecken, ein Einschieben und dergleichen verstanden. Dieser Vorgang soll erfindungsgemäß vorzugsweise werkzeugfrei erfolgen können.

Das lösbare Aufnehmen der Halterung an der medizinischen Vorrichtung findet vorzugsweise an einem Abschnitt des Gehäuses der medizinischen Vorrichtung statt. Dabei ist die Präposition "an" im weiteren Sinne zu verstehen. So kann die Halterung an der medizinischen Vorrichtung auch im Sinne von "in der medizinischen Vorrichtung" oder "auf der medizinischen Vorrichtung" und dergleichen zu verstehen sein.

Der Übergang des zweiten Seitenteils oder eines seiner Abschnitte aus der ersten Stellung in die zweite Stellung kann beispielsweise aufgrund der gebrauchsgemäßen Aufnahme der Halterung an der Vorrichtung erfolgen. Dieser Übergang kann somit durch den Schritt des Einfügens der Halterung an der Vorrichtung bedingt sein. Er kann auch durch das Anordnen als solches der einsetzbaren Einrichtung in das Innere der Halterung bedingt sein. Der Übergang kann dabei - entsprechend der jeweiligen Ausführung der Erfindung - unabhängig davon erfolgen, ob das Einsetzen der Einrichtung in die Halterung und/oder das Einsetzen der Halterung in die medizinischen Vorrichtung in einer vertikalen Richtung, einer horizontalen Richtung oder einer anderen Richtung erfolgt.

Vorteilhafte Weiterbildungen der vorliegenden Erfindung sind jeweils Gegenstand der Unteransprüche.

So weist in einer bevorzugten Ausführungsform ein Abschnitt des zweiten Seitenteils in der ersten Stellung keinen Kontakt zu einer in einem Gebrauchszustand der Halterung im Inneren derselben angeordneten Einrichtung auf. In einer zweiten Stellung steht das zweite Seitenteil oder der Abschnitt hiervon in Kontakt mit der Einrichtung.

Der Kontakt kann erfindungsgemäß insbesondere ein Berührungskontakt oder ein Kraft übertragender Kontakt sein. Es kann sich gegebenenfalls um einen kraftschlüssigen und/oder einen formschlüssigen Kontakt handeln.

Die Einrichtung kann in der zweiten Stellung, der Kontaktstellung, zwischen dem zweiten Seitenteil und einer weiteren Struktur der Halterung, beispielsweise dem ersten Seitenteil, eingeklemmt sein. Die Bewegung des zweiten Seitenteils aus der ersten Stellung in die zweite Stellung oder umgekehrt kann eine Relativbewegung des zweiten Seitenteils zum ersten Seitenteil sein.

Unter einem Kontakt zwischen dem zweiten Seitenteil und der Einrichtung ist erfindungsgemäß nicht ausschließlich ein Berührungskontakt zwischen Seitenteil und Einrichtung im Sinne einer tatsächlichen Berührung benachbarter Strukturen zu verstehen. Vielmehr können zwischen Seitenteil und Einrichtung auch weitere Elemente, wie Abstandshalter, Dämmmaterial, Polsterungen und dergleichen vorgesehen sein. Unter einem "Kontakt" wird im Zusammenhang mit der vorliegenden Erfindung allgemein ein Zustand verstanden, bei welchem das Seitenteil die Einrichtung in ihrer Bewegung maßgeblich beschränkt, und insbesondere ein Zustand, bei welchem mittels des Seitenteils direkt oder indirekt Druck auf einen Abschnitt der Einrichtung ausgeübt wird.

Dabei ist die Halterung in einer weiter bevorzugten erfindungsgemäßen Ausführungsform derart ausgestaltet, dass sich durch gebrauchsgemäßes Einsetzen der Einrichtung in die Halterung von einer ersten Stellung in eine zweite Stellung ein Abstand zwischen dem ersten Seitenteil und dem zweiten Seitenteil oder zwischen Abschnitten jeweils hiervon verringert.

In einer weiter bevorzugten Ausführungsform der erfindungsgemäßen Halterung wird vorgeschlagen, dass das zweite Seitenteil aufgrund der Entnahme der Einrichtung aus dem Inneren der Halterung und/oder aufgrund des Lösens der Halterung von der medizinischen Vorrichtung von der zweiten Stellung (im Folgenden auch "Kontaktstellung" genannt) in die erste Stellung oder in eine Zwischenstellung zwischen der ersten Stellung und der zweiten Stellung übergeht.

Dabei kann der Übergang von der zweiten Stellung in die erste Stellung oder die Zwischenstellung aufgrund von Eigenspannung der Halterung oder des zweiten Seitenteils, durch Einsatz eines Formgedächtnismaterials, beispielsweise durch einen Benutzer der Vorrichtung, eine Rückstell- oder Federeinrichtung oder dergleichen erfolgen.

In einer weiter bevorzugten erfindungsgemäßen Ausführungsform wird vorgeschlagen, dass das zweite Seitenteil in wenigstens einem Abschnitt fest mit der Halterung verbunden ist. Die feste Verbindung kann dabei als eine dauerhafte, als eine nicht lösbare oder gar als eine einstückige Verbindung ausgestaltet sein.

Die Einrichtung, zu deren Aufnahme in ihrem Inneren die Halterung ausgestaltet ist, kann erfindungsgemäß eine Spannungsquelle für die medizinische Vorrichtung sein. Die Einrichtung kann daher als Akku oder Batterie oder dergleichen ausgestaltet sein.

Die Halterung kann erfindungsgemäß einstückig, beispielsweise als Blechstanzteil oder mittels eines Gießverfahrens oder jedem anderen geeigneten Herstellverfahren, unter anderem aus Kunststoff oder dergleichen, ausgestaltet sein.

In einer wiederum weiter bevorzugten erfindungsgemäßen Ausführungsform weist die Halterung wenigstens einen Anschlag zum Festlegen einer Aufnahmetiefe, einer Eintauchtiefe oder dergleichen der Halterung in die medizinische Vorrichtung auf.

Die erfindungsgemäße Halterung weist in einer wiederum weiter bevorzugten Ausführungsform wenigstens eine Rastnase zum Einrasten der Halterung an einem Abschnitt, insbesondere in einer Hinterschneidung, der medizinischen Vorrichtung auf.

In einer wiederum weiter bevorzugten Ausführungsform weist die Halterung wenigstens einen Handgriff zum Tragen der Halterung auf, wobei vorzugsweise mittels Ausübens von Druck auf diesen Handgriff die Rastnase - falls vorhanden - aus dem Einrastzustand an der medizinischen Vorrichtung lösbar ist.

Bei einer wiederum weiter bevorzugten Ausführungsform weist die erfindungsgemäße Halterung wenigstens einen Bodenabschnitt zum Abstützen der Einrichtung in der Halterung auf. Mittels des Bodenabschnitts kann das Gewicht der Einrichtung durch die Halterung getragen werden.

Die erfindungsgemäße Aufgabe wird auch gelöst durch eine medizinische Vorrichtung mit wenigstens einer erfindungsgemäßen Halterung mit Merkmalen des Anspruchs 14. Diese kann vorzugsweise als Dialysevorrichtung ausgestaltet sein. Da die erfindungsgemäßen Vorteile gleichermaßen mit der erfindungsgemäßen Halterung als auch der erfindungsgemäßen Vorrichtung erzielbar sind, wird auf deren gemeinsame, im Wesentlichen unten erfolgende Diskussion verwiesen.

Mittels der vorliegenden Erfindung kann eine Einrichtung wie ein Akku, eine Batterie, insbesondere ein Notstrom-Akku, an einem medizintechnischen Gerät, insbesondere an einer Notfall-Dialysemaschine, unter erhöhter Sicherheit der Haltefunktion befestigt werden. Die Montage bzw. das Befestigen der in der Haltung aufgenommenen Einrichtung an der medizinischen Vorrichtung kann gegenüber Systemen des Standes der Technik stark vereinfacht sein. Ferner kann das Auswechseln der Einrichtung vorteilhaft mit geringem Zeitaufwand verbunden sein. Das Anordnen der Einrichtung an der medizinischen Vorrichtung sowie ihr erneutes Entfernen oder ihr Austauschen kann erfindungsgemäß vorteilhaft ohne Einsatz von Werkzeug erfolgen. Die erfindungsgemäße Halterung kann sich vorteilhaft besonders einfach und kostengünstig herstellen lassen. Das Befestigen der Einrichtung an der medizinischen Vorrichtung sowie das Lösen oder Austauschen der Einrichtung kann erfindungsgemäß ferner vorteilhaft mit einem einzigen Handgriff erfolgen, was wiederum den Bedienkomfort erhöht und die dabei erforderliche Zeitdauer verkürzt.

Die erfindungsgemäße Halterung zeichnet sich vorteilhaft auch dadurch aus, dass sie das Gewicht der Einrichtung in jeder Transportlage sicher abfangen kann. Ferner kann sie den Vorteil aufweisen, dass die Lage der Einrichtung, insbesondere des schweren Akkus, innerhalb der Halterung im Einbauzustand an der medizinischen Vorrichtung vorteilhaft derart unveränderlich vorbestimmt werden kann, dass beispielsweise elektrische Kontaktanschlüsse mit leitenden, nicht für eine Berührung mit den Kontaktanschlüssen vorgesehenen Teilen oder Abschnitten beispielsweise der medizinischen Vorrichtung nicht in Kontakt kommen können. Die vorliegende Halterung kann für Einrichtungen nahezu jeder geometrischen Ausführung einsetzbar sein.

Die vorliegende Erfindung wird im Folgenden anhand der beigefügten Zeichnung exemplarisch beschrieben. Dabei bezeichnen gleiche Bezugszeichen identische oder zumindest ähnliche Elemente. In der Zeichnung gilt:
- Fig. 1: zeigt eine erfindungsgemäße Halterung in einem Nichteinbauzustand ohne eine in einem Inneren der Halterung aufgenommene Einrichtung.
- Fig. 2: zeigt die Halterung der Fig. 1 mit einer hierin aufgenommenen Batterie in einem Einbauzustand in eine medizinische Vorrichtung in einer Ansicht von schräg oben; und
- Fig. 3: zeigt jeweils ausschnittsweise eine in eine erfindungsgemäße medizinische Vorrichtung eingesetzte erfindungsgemäße Halterung.

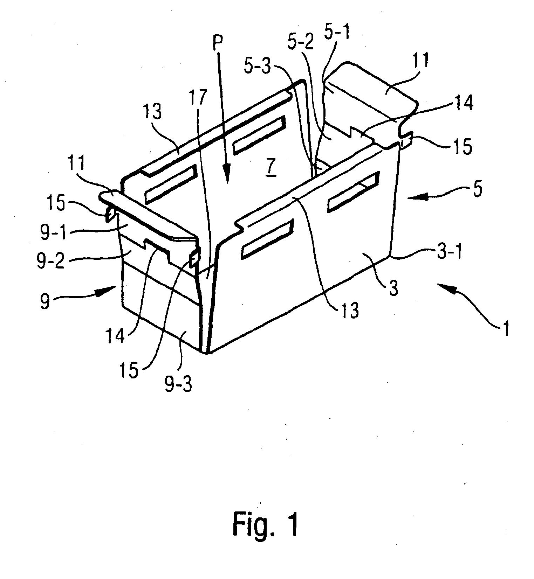

Fig. 1 zeigt in einer Ansicht von schräg oben eine erfindungsgemäße Halterung 1 mit vier Seitenteilen 3, 5, 7 und 9. Die Seitenteile 5, 9 weisen dabei Handgriffe 11 auf, mittels welcher die Halterung 1 leicht transportierbar und auch in eine in Fig. 1 nicht dargestellte medizinische Vorrichtung einsetzbar ist.

Die Seitenteile 3 und 7 sind derart verstellbar zwischen zwei Stellungen ausgestaltet, dass sie durch Auseinanderweichen eine obere Öffnung zwischen zwei Rändern 13 der Seitenteile 3 und 7 im Nicht-Gebrauchszustand der Halterung (d.h. ohne eingesetzte Batterie oder ohne Verbindung mit der medizinischen Vorrichtung) vergrößern und hierdurch das Einsetzen der im Ausführungsbeispiel der Fig. 1 als Batterie ausgestalteten Einrichtung entlang des Pfeils P erleichtern.

Die Seitenteile 5 und 9 können dabei wie im Beispiel der Fig. 1 gezeigt, einen leicht abgestuften Verlauf in einer vertikalen Richtung der Fig. 1 aufweisen, derart, dass obere Abschnitte 5-1 und 9-1 weiter voneinander entfernt stehen als untere Abschnitte 5-3 und 9-3, zwischen welchen jeweils schräge Abschnitte 5-2 und 9-2 liegen. Der stufige Aufbau erlaubt ein Zusammendrücken der Seitenteile 5 und 9 mittels der Handgriffe 11 aufeinander zu derart, dass an den Seitenteilen 5 und 11 vorgesehene Rastnasen 14 auf einfache Weise und ohne den Gebrauch von Werkzeug außer Eingriff mit dem in Fig. 1 nicht dargestellten Gehäuse einer medizinischen Einrichtung - wie weiter unten detaillierter erläutert wird - zu bringen sind.

Die Halterung 1 der Fig. 1 weist ferner vier Anschläge 15 auf, mittels welcher die Aufnahmetiefe oder Eintauchtiefe der Halterung 1 bei ihrem vertikal von oben erfolgenden Einsetzen in das Gehäuse einer in Fig. 1 nicht dargestellten medizintechnischen Vorrichtung begrenzt ist. Die Halterung 1 kann somit mittels der Anschläge 15 im Gehäuse der medizinischen Vorrichtung abgestützt ruhen.

Die Seitenteile 3 und 7 nähern sich im eingebauten Zustand der Halterung 1 zumindest in ihren oberen Bereichen aufeinander zu. Bei erneuter Entnahme der Halterung 1 aus der medizintechnischen Vorrichtung weichen sie wieder auseinander. Die Halterung kann jedoch auch derart ausgestaltet sein, dass sich die Seitenteile 3 und 7 durch Entnahme der in Fig. 1 nicht dargestellten Batterie in Abschnitten hiervon voneinander weg bewegen.

Wie in Fig. 1 gezeigt ist, weist die Halterung der dort dargestellten erfindungsgemäßen Ausführungsform einen Bodenabschnitt 17 auf, mit welchem die Seitenteile 3, 5, 7 und 9 verbunden sind. Erfindungsgemäß ist es jedoch auch möglich, die Halterung 1 ohne einen solchen Bodenabschnitt 17 auszugestalten. Bevorzugt sind alternativ zum Bodenabschnitt 17 in solchen Ausführungsformen Halte- oder Stützabschnitte zum Tragen der in das Innere der Halterung 1 einzusetzenden Einrichtung beispielsweise an den Seitenwänden vorgesehen.

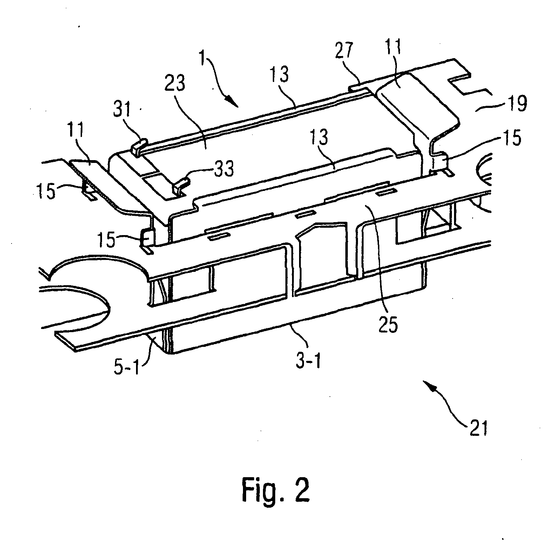

Fig. 2 zeigt die erfindungsgemäße Halterung 1 der Fig. 1 in einem Einbauzustand in einem Gehäuse 19 einer medizinischen Vorrichtung 21. Die Halterung 1 weist in der Darstellung der Fig. 2 in ihrem Inneren eine Einrichtung 23 in Form einer Batterie mit Kontakten 25 und 27 auf.

In Fig. 2 ist zu erkennen, wie sich die Halterung 1 mittels der Anschläge 15 an einem oberen Bereich des Gehäuses 19 der medizinischen Vorrichtung 21 abstützt und wie diese somit festlegen, wie weit die vertikal von oben in die medizinische Vorrichtung 21 eingesetzte Halterung 1 in diese eintaucht bzw. eindringt.

In Fig. 2 ist zu erkennen, wie die Seitenteile 3, 5 aufgrund der Begrenzung der Öffnungsweite für die Halterung 1 im Gehäuse 19 aufgrund von Gehäuseabschnitten 25 und 27 bei ihrem Einsetzen derart aufeinander zu bewegt wurden, dass die Abschnitte bzw. Ränder 13 die Einrichtung 23 von oben umschlingen oder von der Seite her festklemmen und in ihrer Bewegung einschränken und somit befestigen bzw. sichern.

Eine Umschlingung des oberen Bereichs der Batterie mit Kontakten 31 und 33 bzw. der Einrichtung 23, also im Bereich ihrer Kontaktseite, ist somit gegeben.

In einem unteren Abschnitt 3-1, welcher beispielsweise als Falz ausgestaltet ist, ist das Seitenteil 3 mit dem Bodenabschnitt 17 in der Ausführungsform der Fig. 1 und 2 einstückig verbunden. Dasselbe kann ebenso für die Seitenteile 5, 7 und 9 gelten.

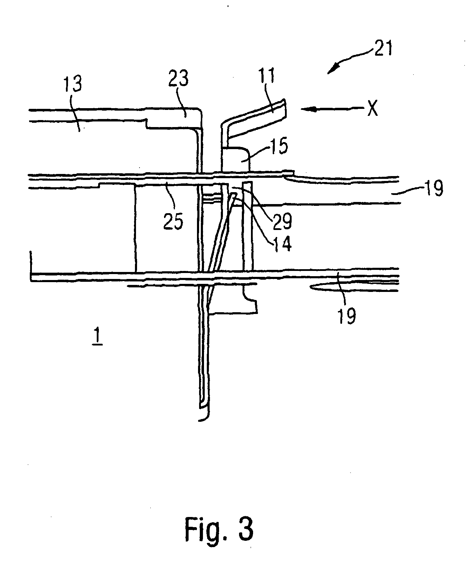

Fig. 3 stellt in einer leicht gekippten Seitenansicht ausschnittsweise eine erfindungsgemäße Halterung 1 in ihrer Anordnung im Gehäuse 19 der Vorrichtung 21 dar. In Fig. 3 ist die Rastnase 14 der erfindungsgemäßen Halterung 1 zu erkennen, welche aufgrund von Eigenspannung des Seitenteils 5, welches die Rastnase 14 trägt, in Eingriff steht mit einem Abschnitt 29 des Gehäuses 19, derart, dass ein Lösen der Halterung 1 aus dem Gehäuse 19 der medizinischen Vorrichtung 21 nach oben - d.h. in einer vertikalen Richtung - verhindert wird. Durch ein Drücken des Handgriffs 11 in der mit dem Pfeil X bezeichneten Richtung ist ein Lösen bzw. Entrasten der Rastnase 14 vom Abschnitt 29 des Gehäuses 19 zum Entnehmen der Halterung 1 aus der medizinischen Vorrichtung 21 möglich.

## Patentansprüche

1. Halterung (1) zum lösbaren Aufnehmen derselben an einer medizinischen Vorrichtung (21) und zum Aufnehmen einer in ein Inneres der Halterung (1) einsetzbaren Einrichtung (23), wobei die Halterung (1) aufweist:
wenigstens zwei Seitenteile (3, 7) mit einem ersten Seitenteil (3) und einem zweiten Seitenteil (7), wobei
wenigstens das zweite Seitenteil (7) oder wenigstens ein Abschnitt (13) hiervon ausgestaltet ist, um durch gebrauchsgemäßes Anordnen der Halterung (1) an der medizinischen Vorrichtung (21) unter Verringerung eines Volumens des inneren Abschnitts von einer ersten Stellung in eine zweite Stellung überzugehen und/oder durch gebrauchsgemäßes Einsetzen der Einrichtung (23) in die Halterung (1) unter Verringerung eines Volumens des inneren Abschnitts von einer ersten Stellung in eine zweite Stellung überzugehen.

2. Halterung (1) nach Anspruch 1, wobei wenigstens das zweite Seitenteil (7) oder wenigstens ein Abschnitt (13) hiervon in der ersten Stellung keinen Kontakt zu der gebrauchsgemäß in das Innere der Halterung (1) eingesetzten Einrichtung (23) hat, und in der zweiten Stellung Kontakt mit der Einrichtung (23) hat.

3. Halterung (1) nach einem der Ansprüche 1 oder 2, wobei das zweite Seitenteil (7) ausgestaltet ist, um unter Verringerung eines Abstandes zwischen dem ersten Seitenteil (3) und dem zweiten Seitenteil (7) oder zwischen Abschnitten jeweils hiervon von der ersten Stellung in die zweite Stellung überzugehen.

4. Halterung (1) nach einem der vorangegangenen Ansprüche, wobei die Halterung (1) derart ausgestaltet ist, dass das zweite Seitenteil (7) aufgrund der Entnahme der Einrichtung (23) aus dem Inneren der Halterung (1) und/oder aufgrund des Lösens der Halterung (1) von der medizinischen Vorrichtung (21) aus der zweiten Stellung in die erste Stellung oder in eine Zwischenstellung zwischen der ersten Stellung und der zweiten Stellung übergeht.

5. Halterung (1) nach einem der vorangegangenen Ansprüche, wobei das zweite Seitenteil (7) in wenigstens einem Abschnitt fest mit der Halterung (1) verbunden ist.

6. Halterung (1) nach einem der vorangegangenen Ansprüche, wobei die Einrichtung (23) eine Spannungsquelle für die medizinische Vorrichtung (21) ist.

7. Halterung (1) nach einem der vorangegangenen Ansprüche, wobei die Halterung (1) ausgestaltet ist für ihre Anordnung in einer Aussparung eines Gehäuses (19) der medizinischen Vorrichtung (21).

8. Halterung (1) nach einem der vorangegangenen Ansprüche, wobei die Halterung (1) einstückig ausgestaltet ist.

9. Halterung (1) nach einem der vorangegangenen Ansprüche mit wenigstens einem Anschlag (15) zum Festlegen einer Aufnahmetiefe der Halterung (1) in die medizinische Vorrichtung (21).

10. Halterung (1) nach einem der vorangegangenen Ansprüche mit wenigstens einer Rastnase (14) zum Einrasten an einem Abschnitt (29) der medizinischen Vorrichtung (21) zum Erzeugen eines Einrastzustandes.

11. Halterung (1) nach Anspruch 10 mit wenigstens einem Handgriff (11), mittels welchem die Rastnase (14) aus dem Einrastzustand lösbar ist.

12. Halterung (1) nach einem der vorangegangenen Ansprüche mit wenigstens einem Bodenabschnitt (17).

13. Halterung (1) nach einem der vorangegangenen Ansprüche mit wenigstens einer Einrichtung (23).

14. Medizinische Vorrichtung (21) mit wenigstens einer Halterung (1) nach einem der Ansprüche 1 bis 13.

15. Medizinische Vorrichtung (21) nach Anspruch 14, welche als Dialysevorrichtung ausgestaltet ist.

## Claims

1. A mount (1) for being releasably received on a medical apparatus (21) and for receiving a device (23) adapted for being inserted into the interior of the mount (1), said mount (1) comprising:
at least two side parts (3, 7) including a first side part (3) and a second side part (7), wherein
at least the second side part (7) or at least a portion (13) thereof is configured so as to pass from a first position into a second position as a result of operational arrangement of the mount (1) on the medical apparatus (1) while reducing a volume of the interior and/or as a result of operational insertion of the device (23) into the mount (1) while reducing a volume of the interior.

2. The mount (1) according to claim 1, wherein at least the second side part (7) or at least a portion (13) thereof is not in contact with the device (23) operationally inserted into the interior of the mount (1) when in the first position, and is in contact with the device (23) in the second position.

3. The mount (1) according to any one of claims 1 or 2, wherein the second side part (7) is configured so as to pass from the first position into the second position while reducing a distance between the first side part (3) and the second side part (7) or between respective portions thereof.

4. The mount (1) according to any one of the preceding claims, wherein the mount (1) is configured such that the second side part (7) passes from the second position into the first position or into an intermediary position between the first position and the second position owing to removal of the device (23) from the interior of the mount (1) and/or owing to release of the mount (1) from the medical apparatus (21).

5. The mount (1) according to any one of the preceding claims, wherein the second side part (23) is fixedly connected to the mount (1) at least in a portion thereof.

6. The mount (1) according to any one of the preceding claims, wherein the device (23) is a power source for the medical apparatus (21).

7. The mount (1) according to any one of the preceding claims, wherein the mount (1) is configured for being arranged inside a recess of a casing (19) of the medical apparatus (21).

8. The mount (1) according to any one of the preceding claims, wherein the mount (1) has an integral configuration.

9. The mount (1) according to any one of the preceding claims, comprising at least one stop (15) for fixing a depth of reception of the mount (1) in the medical apparatus (21).

10. The mount (1) according to any one of the preceding claims, comprising at least one catch (14) for latching on a portion (29) of the medical apparatus (21) in order to produce a latched condition.

11. The mount (1) according to claim 10, comprising at least one handle (11) whereby the catch (14) may be released from the latched condition.

12. The mount (1) according to any one of the preceding claims, comprising at least one bottom portion (17).

13. The mount (1) according to any one of the preceding claims, comprising at least one device (23).

14. A medical apparatus (21) comprising at least one mount (1) according to any one of claims 1 to 13.

15. The medical apparatus (21) according to claim 14, which has the form of a dialysis apparatus.

## Revendications

1. Support (1) pouvant être reçu de manière libérable sur un système médical (21) et susceptible de recevoir d'un dispositif (23) pouvant être introduit à l'intérieur du support (1), dans lequel le support (1) présente les éléments suivants:
au moins deux parties latérales (3, 7) avec une première partie latérale (3) et une seconde partie latérale (7), dans lequel
au moins la seconde partie latérale (7) ou au moins un tronçon (13) de celle-ci est conçue pour passer d'une première position à une seconde position, grâce à l'agencement conforme à l'utilisation du support (1) sur le système médical (21) en réduisant le volume de l'intérieur et/ou pour passer d'une première position à une seconde position, grâce à l'introduction conforme à l'utilisation du dispositif (23) dans le support (1) en réduisant le volume de l'intérieur.

2. Support (1) selon la revendication 1, dans lequel au moins la seconde partie latérale (7) ou au moins un tronçon (13) de celui-ci, dans la première position, n'a aucun contact avec le dispositif (23) introduit conformément à l'utilisation à l'intérieur du support (1) et dans la seconde position, fait contact avec le dispositif (23).

3. Support (1) selon l'une quelconque des revendications 1 ou 2, alors que la seconde partie latérale (7) est conçue pour passer respectivement de la première position à la seconde position en réduisant l'écartement entre la première partie latérale (3) et la seconde partie latérale (7) ou entre des segments de celle-ci.

4. Support (1) selon l'une des revendications précédentes, dans lequel le support (1) est conçue de telle sorte que la seconde partie latérale (7) après avoir retiré le dispositif (23) de l'intérieur du support (1) et/ou après avoir desserré le support (1) du système médical (21) passe de la seconde position dans la première position ou bien dans une position intermédiaire entre la première position et la seconde position.

5. Support (1) selon l'une des revendications précédentes, tandis que la seconde partie latérale (7) est solidement reliée au support (1) par le biais d'au moins un tronçon.

6. Support (1) selon l'une des revendications précédentes, dans lequel le dispositif (23) est une source de tension pour le système médical (21).

7. Support (1) selon l'une des revendications précédentes, tandis que le support (1) est conçu pour son agencement dans le retrait d'une boîtier (19 du système médical (21).

8. Support (1) selon l'une des revendications précédentes, dans lequel le support (1) est conçu d'un seul tenant.

9. Support (1) selon l'une des revendications précédentes, avec au moins une butée (15) pour définir une profondeur de réception du support (1) dans le système médical (21).

10. Support (1) selon l'une des revendications précédentes, pourvu au moins d'un nez d'encliquetage (14) pour s'encliqueter sur un tronçon (29) du système médical (21) pour créer un état d'encliquetage.

11. Support (1) selon la revendication 10 avec au moins une poignée (11), au moyen de laquelle le nez d'encliquetage (14) peut être libéré de l'état d'encliquetage.

12. Support (1) selon l'une des revendications précédentes, comportant au moins un tronçon de fond (17).

13. Support (1) selon l'une des revendications précédentes, comportant au moins un dispositif (23).

14. Système médical (21) pourvu au moins d'un support (1) selon l'une des revendications 1 à 13.

15. Système médical (21) selon la revendication 14, qui est conçu comme équipement de dialyse.
